# EUROPÄISCHE PATENTANMELDUNG

(11) **EP 0 621 259 A1**
(43) Veröffentlichungstag der Anmeldung: **26.10.1994**
(21) Anmeldenummer: 94105692.1
(22) Anmeldetag: 13.04.1994
(51) Int. Cl.: C07C 209/36, C07C 211/52, C07C 245/20, G03C 1/54

(54) **Verfahren zur Herstellung von aromatischen Aminen und Diazoniumsalzen**

(30) Priorität: 20.04.1993 DE 4312708
(71) Anmelder: HOECHST AKTIENGESELLSCHAFT, D-65929 Frankfurt am Main (DE)
(72) Erfinder: Beller, Matthias, Dr., D-65527 Niedernhausen (DE); Strutz, Heinz, Dr., D-61250 Usingen (DE)

(57) **Zusammenfassung**

Ein Verfahren zur Herstellung halogenierter aromatischer Amine durch Reduktion der entsprechenden Nitroverbindungen mit Hydrazin in Gegenwart eines heterogenen Katalysators, der ein Metall aus der Gruppe VIIIa enthält, dadurch gekennzeichnet, daß die Umsetzung
a) in einem Temperaturbereich von etwa -20°C bis etwa 70°C mit
b) etwa 1,0 bis etwa 1,7 Äquivalenten Hydrazin, bezogen auf die zu reduzierende(n) Nitrogruppe(n),
c) in Gegenwart eines heterogenen Palladiumkatalysators,
durchgeführt wird.

Das Verfahren liefert halogenierte aromatische Amine in guter Ausbeute ohne Dehalogenierungsreaktionen.

Gegebenenfalls kann in situ direkt zu Diazoniumsalzen umgesetzt werden.

## Beschreibung

Die Erfindung betrifft ein Verfahren zur Herstellung halogenierter aromatischer Amine und Diazoniumsalze durch katalysierte Transferhydrierung halogenierter Nitroaromaten und, gegebenenfalls, anschließende in situ Diazotierung.

Die Wasserstoff-Transferhydrierung stellt eine interessante Alternative zu den klassischen Hydrierverfahren, die mit molekularem Wasserstoff arbeiten, dar. Bei dieser Methode wird Wasserstoff von einem organischen Donormolekül, wie Cyclohexen, Ameisensäure oder Hydrazin, auf ein Substrat, z. B. ein Olefin oder eine Nitroverbindung, übertragen. Das Donormolekül muß dabei über ein genügend tiefes Oxidationspotential verfügen, damit die Wasserstoffübertragung unter milden Bedingungen ablaufen kann. Im allgemeinen wird die Reaktion in Gegenwart eines Katalysators, der üblicherweise ein Übergangsmetall enthält, durchgeführt.
Übersichtsartikel zur Wasserstoff-Transferhydrierung finden sich beispielsweise in Chem. Rev. 85 (1985) 129-170; Chem. Rev. 74 (1974) 567-580, Chem. Rev. 92 (1992) 1051-1069 und Comprehensive Organic Synthesis; Ed. B.M. Trost, I. Fleming, Pergamon Press 1991; Volume 8.

Die einfache Durchführung und die Vermeidung von Sicherheitsproblemen, die mit der Handhabung von Druckapparaturen einhergehen, machen die Wasserstoff-Transferreaktionen auch industriell interessant. Trotzdem besitzen Wasserstoff-Transferverfahren derzeit kaum technische Bedeutung, was durch die bislang niedrigen Raum-Zeit-Ausbeuten und die nicht immer ausreichende Chemoselektivität der bisher bekannten Verfahren bedingt ist.

Die Reduktion von aromatischen Nitroverbindungen durch Transferhydrierung mit Hydrazin ist seit langem bekannt (siehe z. B. A. Furst et al., Chem. Rev. 65 (1965) 5).

Bei halogenierten Nitroaromaten, insbesondere bei Chlor und Bromverbindungen, verläuft die Reaktion jedoch unter Dehalogenierung.
So beschreibt W. L. Mosby (Chemistry and Industry 1959, 1348 und J. Org. Chem. 24 (1959), 421) beispielsweise die reduktive Dehalogenierung von Bromonitrophenanthren in siedendem Ethanol.

Auch in dem oben zitierten Übersichtsartikel von A. Furst et al. wird deutlich gemacht, daß Palladiumkatalysatoren in Gegenwart von Hydrazin effektiv die reduktive Dehalogenierung halogenierter aromatischer Nitroverbindungen katalysieren. So werden Palladiumverbindungen als spezifische Dehalogenierungskatalysatoren beschrieben. Pietra (Chem. Rev. 65 (1965) 51) demonstrierte, daß die Reduktion von Bromnitrobenzolen unter Palladiumkatalyse lediglich Anilin und nicht Bromaniline liefert. Busch und Schmidt (Chem. Ber. 62 (1929) 2612) dehalogenierten eine Reihe von Halogenbenzolen mit Hydrazin und Palladium auf Calciumcarbonat.

Noch im Jahre 1991 wird Palladium auf Kohlenstoff als ein Katalysator beschrieben, der bei Transferhydrierungsreaktionen zur Dehalogenierung verwendet werden kann (siehe z.B. G. W. Kabalka und R. S. Varma in: Comprehensive Organic Synthesis, Vol. 8, 363-380, Pergamon Press 1991).

Somit läßt sich feststellen, daß das Reduktionssystem Palladiumkatalysator/Hydrazin dem Fachmann als wirksames Dehalogenierungssystem bekannt ist, das im Falle halogenierter aromatischer Nitroverbindungen Dehalogenierungsreaktionen katalysiert.

Raney-Nickel führt bei der Reduktion halogenierter Nitroaromaten nicht zu einheitlichen Produkten (siehe z. B. die unterschiedlichen Ergebnisse von Leggetter und Brown, Can. J. Chem. 38 (1960) 2363 und Müller und Weisbrod, J. prakt. Chem. 111 (1925) 307); daher war man bisher zur Durchführung dieser Reaktion auf teuere und toxikologisch wie ökologisch bedenkliche Katalysatormetalle, wie Ruthenium, Osmium und Rhodium (siehe z. B. M. Busch et al., J. prakt. Chem., 146 (1936) 1 und S. Pietra, Ann. Chim. (Rom) 47 (1957) 410), angewiesen.

Es bestand demnach ein erhebliches Bedürfnis nach einem Verfahren, das die selektive Reduktion aromatischer Nitroverbindungen unter Katalyse durch Palladium bei einfacher technischer Umsetzung ermöglicht.

Insbesondere sollte ein Verfahren gefunden werden, bei welchem ein einfaches stabiles Katalysatorsystem verwendet werden kann, das technisch rückgewinnbar ist, und das es erlaubt, das hergestellte halogenierte Anilin ohne weitere Aufarbeitung direkt zu Diazoniumsalzen umzusetzen.

Es wurde nun überraschend gefunden, daß heterogene Palladiumkatalysatoren mit Hydrazin als Reduktionsmittel hochselektiv aromatische Nitroverbindungen reduzieren, ohne dabei Halogenatome reduktiv zu dehalogenieren, wenn die Reaktion in einem bestimmten Temperaturbereich und mit einem bestimmten Verhältnis von Hydrazin zu der zu reduzierenden Nitroverbindungen durchgeführt wird.

Gegenstand der Erfindung ist daher ein Verfahren zur Herstellung halogenierter aromatischer Amine durch Reduktion der entsprechenden Nitroverbindungen mit Hydrazin in Gegenwart eines heterogenen Katalysators, der ein Metall aus der Gruppe VIIIa enthält, dadurch gekennzeichnet, daß die Umsetzung
a) in einem Temperaturbereich von etwa -20°C bis etwa 70°C mit
b) etwa 1,0 bis etwa 1,7 Äquivalenten Hydrazin, bezogen auf die zu reduzierende(n) Nitrogruppe(n),
c) in Gegenwart eines heterogenen Palladiumkatalysators,
durchgeführt wird.

Das erfindungsgemäße Verfahren ermöglicht es, halogenierte aromatische Amine selektiv und in hohen Ausbeuten darzustellen. Die Verwendung von Palladium als Katalysatormetall hat ökonomische und ökologische Vorteile. Ein weiterer Vorteil des erfindungsgemäßen Verfahrens liegt darin, daß die aromatischen Amine nach dem Abfiltrieren des Katalysators direkt in hohen Ausbeuten zu den entsprechenden Diazoniumsalzen diazotiert werden können. Damit sind auch aromatische Diazoniumsalze, insbesondere halogenierte Diazoniumsalze technisch besser als bisher zugänglich. Weiterhin vorteilhaft ist es, daß das Katalysatorsystem heterogen vorliegt und somit durch einfaches Abfiltrieren ein Recycling des wertvollen Palladiumkatalysators möglich ist. Die milden Reaktionsbedingungen verhindern zudem eine Deaktivierung des Katalysators.

Als Ausgangsverbindungen in dem erfindungsgemäßen Verfahren dienen aromatische Nitroverbindungen, die mindestens noch ein Halogenatom, vorzugsweise Chlor, Brom oder Iod, besonders bevorzugt Chlor oder Brom, enthalten.
Beispiele sind entsprechend substituierte Benzole, Naphthaline, Phenanthrene und Heterocyclen.

Bevorzugte Ausgangsverbindungen sind solche der Formel I, 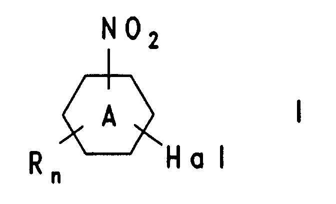
worin die Symbole und Indizes folgende Bedeutung haben: 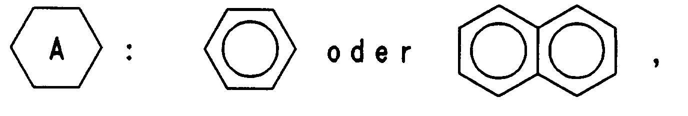
- Hal:: -F, -Cl, -Br, -I, vorzugsweise -Cl, -Br, -I, besonders bevorzugt -Cl, -Br,
- n:: je nach aromatischem Grundkörper 0, 1, 2, 3, 4 oder 0, 1, 2, 3, 4, 5, 6,
- R:: gleich oder verschieden -H, -F, -Cl, -Br, -I, CN, -SCN, -OCN, -NCO, -OH, -NH₂, -CHO, -NO₂, -SO₃H, R', OR', -COOR', -OCOR', -NHR', -NR'₂, -NA'H, -NA'R', -NA'₂, NHCOR', NHCOA',
- R':: gleich oder verschieden eine verzweigte oder unverzweigte Alkyl-, Alkenyl- oder Alkinylgruppe mit 1 bis 12, vorzugsweise 1 bis 8, Kohlenstoffatomen, bei der eine oder mehrere CH₂-Gruppen durch -O- und ein oder mehrere H-Atome durch -F, -Cl, -Br, -I, -OH und/oder -NH₂ ersetzt sein können,
- A':: Phenyl und/oder Naphthyl, die beide gegebenenfalls substituiert sein können.

Bevorzugt sind Verbindungen in denen
- n: 0, 1 oder 2 ist und
- R: eine Methyl-, Ethyl-, n-Propyl-, Isopropyl-, n-Butyl-, tert.-Butyl-, n-Pentyl-, i-Pentyl-, n-Hexyl-, n-Heptyl-, n-Octyl-, 2-Ethylhexyl-, 2-Ethyldecyl-, n-Decyl-, n-Dodecyl-, ferner eine Methoxy-, Ethoxy-, Propoxy-, Isopropoxy-, Butoxy-, Pentyloxy-, Hexyloxy-, Octyloxy-, 2-Ethylhexyloxy-, Decyloxy-, Dodecyloxy-, ferner eine Carbonsäure-, Carbonsäuremethylester-, Carbonsäureethylester-, Carbonsäurepropylester-, Carbonsäurebutylester-, Carbonsäurepentylester-, Carbonsäurehexylester-, Carbonsäure-2-ethylhexylester-, Carbonsäurephenylester-, Formyl-, Carbonsäureamid-, Carbonsäuredimethylamid-, Carbonsäurediethylamid-, Carbonsäuremethylamid-, Carbonsäureethylamid-, ferner eine N,N-Dimethylamino-, N,N-Diethylamino-, N-Methylamino-, N-Ethylamino-, N,N-Methylethylamino-, N,N-Dipropylamino-, N,N-Dibutylamino-, N-Morpholino-, schließlich eine Acetoxy-, Propionyloxy- und Butyryloxygruppe und/oder eine Phenylgruppe, die ebenfalls substituiert sein kann.

Als Reduktionsmittel werden Hydrazin, Hydrazinderivate, wie Methylhydrazin, oder Hydraziniumsalze, wie Hydraziniumchlorid und Hydraziniumsulfat eingesetzt.
Das Reduktionsmittel kann in reiner Form oder als Lösung, beispielsweise als wäßrige, alkoholische oder etherische Lösung, verwendet werden. Bevorzugt ist der Einsatz einer 60 - 80 gew.-%igen Lösung von Hydrazin in Wasser. Das Katalysatormetall Palladium ist vorzugsweise auf einem Trägermaterial, wie Aktivkohle, Calciumcarbonat, Bariumsulfat, Bimsstein, Tonerde, Kieselgur, Graphit, Kieselgel, Magnesiumoxid und/oder Aluminiumoxid aufgebracht. Besonders bevorzugt ist das Katalysatormetall Palladium auf Aktivkohle und/oder Aluminiumoxid und/oder Kieselgel, insbesondere bevorzugt auf Aktivkohle, aufgebracht.

Es ist bevorzugt, einen heterogenen Palladiumkatalysator anzuwenden, der etwa 0,5 bis 20 Gew.-%, besonders bevorzugt 1 bis 10 Gew.-%, insbesondere bevorzugt 5 Gew.-% Palladium, bezogen auf das Trägermaterial, enthält.

Hinsichtlich des Mengenverhältnisses Palladium zu Nitroaromat ist es zweckmäßig, etwa 0,001 bis etwa 20 mol-%, vorzugsweise etwa 0,05 bis etwa 5 mol-% Palladium, bezogen auf den Nitroaromaten, anzuwenden.

Es werden pro Nitrogruppe im Nitroaromaten etwa 1,0 bis 1,7 mol, vorzugsweise 1,2 bis 1,5 mol Hydrazin zum Einsatz gebracht.

Das erfindungsgemäße Verfahren wird vorzugsweise in einem Lösungsmittel, in der Regel in einem organischen Lösungsmittel, wie einem cyclischen oder acyclischen Ether oder einem Alkohol, durchgeführt. Es kann aber auch bei bestimmten Nitroaromaten ohne Lösungsmittel oder in Wasser durchgeführt werden.

Besonders bevorzugt sind als Lösungsmittel Tetrahydrofuran, Methanol, Ethanol, Isopropanol, Dioxan und Ethylenglykol.

Das erfindungsgemäße Verfahren wird bei Temperaturen von etwa -20°C bis etwa 70°C, vorzugsweise von 0°C bis 60°C, besonders bevorzugt von 0°C bis 50°C, insbesondere bevorzugt von 0°C bis 40°C, durchgeführt.

In einer bevorzugten Variante des erfindungsgemäßen Verfahrens wird die halogenierte, aromatische Nitroverbindung, vorzugsweise gelöst in einem Lösungsmittel, vorgelegt und mit der gewünschten Menge Katalysator versetzt. Anschließend gibt man unter Rühren und Kühlen das Reduktionsmittel, beispielsweise gelöst in Wasser, so zu, daß die gewünschte Innentemperatur nicht überschritten wird. Vorzugsweise wird das Reduktionsmittel über einen Zeitraum von 5 Minuten bis 12 Stunden zugegeben. Nach Zugabe des Reduktionsmittels wird bei einer Temperatur, die gegebenenfalls gegenüber der bei der Zugabe eingestellten erhöht sein kann, über einen Zeitraum von 5 Minuten bis 48 Stunden, vorzugsweise 0,5 Stunden bis 12 Stunden, nachgerührt.

Die Reaktion wird bei oxidationsempfindlichen Produkten vorzugsweise unter einer Schutzgasatomosphäre, beispielsweise unter Stickstoff, durchgeführt.

Die Aufarbeitung erfolgt nach bekannten, dem Fachmann geläufigen Methoden. Beispielsweise kann man den Katalysator abfiltrieren, das Lösungsmittel abziehen und das so erhaltene Rohprodukt durch Umkristallisation oder Chromatographie weiter aufreinigen. In einer bevorzugten Variante wird der Katalysator abfiltriert und die so erhaltene Lösung direkt weiter umgesetzt. Nach dem erfindungsgemäßen Verfahren bevorzugt herstellte Verbindungen sind solche der Formel II 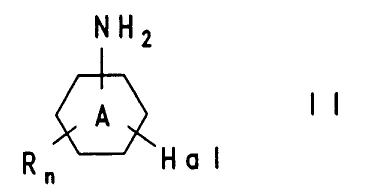
in der die Symbole und Indizes folgende Bedeutung haben: 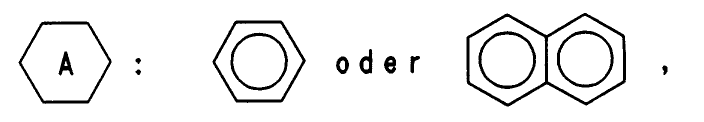
- Hal:: -F, -Cl, -Br, -I, vorzugsweise -Cl, -Br, -I, besonders bevorzugt -Cl, -Br,
- n:: je nach aromatischem Grundkörper 0, 1, 2, 3, 4 oder 0, 1, 2, 3, 4, 5, 6,
- R:: gleich oder verschieden -H, -F, -Cl, -Br, -I, CN, -SCN, -OCN, -NCO, -OH, -NH₂, -CHO, -SO₃H, R', OR', -COOR', -OCOR', -NHR', -NR'₂, -NA'H, -NA'R', -NA'₂, NHCOR', NHCOA',
- R':: gleich oder verschieden eine verzweigte oder unverzweigte Alkyl-, Alkenyl- oder Alkinylgruppe mit 1 bis 12, vorzugsweise 1 bis 8, Kohlenstoffatomen, bei der auch eine oder mehrere CH₂-Gruppen durch -O- und ein oder mehrere H-Atome durch -F, -Cl, -Br, -I, -NH₂ und/oder -OH ersetzt sein können,
- A':: Phenyl und/oder Naphthyl, die beide gegebenenfalls substituiert sein können.

Die erfindungsgemäß hergestellten Verbindungen finden vielfältige Verwendung, beispielsweise als Zwischenprodukte zur Herstellung von Wirkstoffen, Polymeren und Farbstoffen.

Bevorzugt dienen die erfindungsgemäß hergestellten Verbindungen als Ausgangsstoffe zur Herstellung von Diazoniumsalzen.

Dabei ist es zweckmäßig, die erfindungsgemäß hergestellten Verbindungen nicht zu isolieren, sondern nach Abfiltrieren des Katalysators direkt in der Reaktionslösung weiter umzusetzen. Die Herstellung der Diazoniumsalze kann nach dem Fachmann bekannten Methoden erfolgen, wie sie beispielsweise in Houben-Weyl, Methoden der Organischen Chemie, 4. Aufl. Band 10/3, S. 1-212, Georg Thieme Verlag, 1965, beschrieben sind.
In einer bevorzugten Variante wird die Reaktionslösung auf eine Temperatur von -10°C bis +5°C gekühlt und mit einer Säure, wie Tetrafluoroborsäure, im Überschuß versetzt. Anschließend wird mit einer wäßrigen Lösung von Natriumnitrit versetzt, nachgerührt und das ausgefallene Diazoniumsalz abfiltriert und getrocknet.

Gegenstand der Erfindung ist daher auch ein Verfahren zur Herstellung von halogenierten Diazoniumsalzen, dadurch gekennzeichnet, daß
a) eine halogenierte aromatische Nitroverbindung, gegebenenfalls in einem Lösungsmittel, bei einer Temperatur zwischen etwa -20°C und etwa 70°C mit 1,0 bis 1,7 Äquivalenten Hydrazin, bezogen auf die zu reduzierende(n) Nitrogruppe(n), in Gegenwart eines heterogenen Palladiumkatalysators umgesetzt wird,
b) der Katalysator abfiltriert wird,
c) die Reaktionslösung bei -10°C bis 15°C mit einem Überschuß einer Säure und anschließend mit einer wäßrigen Lösung von Natriumnitrit versetzt wird,
d) gegebenenfalls nachgerührt wird und
e) das entstandene Diazoniumsalz abfiltriert wird.

Vorzugsweise werden mit dem erfindungsgemäßen Verfahren Diazoniumsalze der Formel III hergestellt, 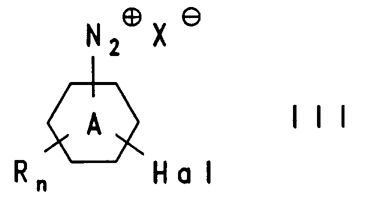
worin die Symbole und Indizes folgende Bedeutung haben: 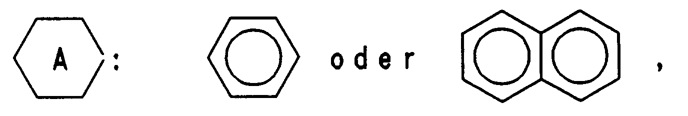
- Hal:: -F, -Cl, -Br, -I, vorzugsweise -Cl, -Br, -I, besonders bevorzugt -Cl, -Br,
- n:: je nach aromatischem Grundkörper 0, 1, 2, 3, 4 oder 0, 1, 2, 3, 4, 5, 6,
- R:: gleich oder verschieden -H, -F, -Cl, -Br, -I, CN, -SCN, -OCN, -NCO, -OH, -CHO, NO₂, -SO₃H, R', OR', -COOR', -OCOR', -NHR', -NR'₂, -NA'H, -NA'R', -NA'₂, NHCOR', NHCOA',
- R':: gleich oder verschieden eine verzweigte oder unverzweigte Alkyl-, Alkenyl- oder Alkinylgruppe mit 1 bis 12, vorzugsweise 1 bis 8, Kohlenstoffatomen, bei der auch eine oder mehrere CH₂-Gruppen durch -O- und ein oder mehrere H-Atome durch -F, -Cl, -Br, -I,-OH und/oder -NH₂ ersetzt sein können,
- A':: Phenyl und/oder Naphthyl, die beide gegebenenfalls substituiert sein können,
- X:: Cl, BF₄, PF₆, HSO₄, SO₄⁻, Tosylat, 5-Sulfosalicylat.

Die erfindungsgemäß hergestellten Diazoniumsalze dienen beispielsweise als Zwischenprodukte zur Herstellung von Wirkstoffen, Polymeren und Farbstoffen und insbesondere als Diazotypievorprodukte.

Die Erfindung wird durch die Beispiele näher erläutert.

In den Beispielen verhalten sich Gewichtsteile (GT) zu Volumenteilen (VT) wie Gramm zu Milliliter.

### Beispiel 1 2-Chlor-4-N,N-dimethylaminoanilin

100,0 GT 2-Chlor-N,N-dimethyl-4-nitroanilin werden bei Raumtemperatur in 280 VT Tetrahydrofuran gelöst. Dazu gibt man 10,0 GT Palladium auf Aktivkohle (5 Gew-%ig). Zu dem Reaktionsgemisch tropft man innerhalb von 40 Minuten 42,9 VT einer 80 gew-%igen wäßrigen Hydrazinlösung unter guter Kühlung so zu, daß eine Innentemperatur zwischen 15 und 21°C nicht überschritten wird. Anschließend wird eine Stunde bei Raumtemperatur gerührt. Die gaschromatographische Analyse der Reaktionsmischung zeigt bei 100 % Umsatz 94,5 % Produkt und 4,2 % dechloriertes Diamin (Ausbeute: 94,5 % 2-Chlor-4-N,N-dimethylaminoanilin).

### Beispiel 2 2-Chlor-4-N,N-dimethylaminophenyldiazoniumtetrafluoroborat

Es wird die Reaktionslösung aus Beispiel 1 verwendet. Der Katalysator wird abfiltriert und mit 50 VT Tetrahydrofuran gewaschen. Das Filtrat wird anschließend auf 0°C abgekühlt und mit 216 VT 50 gew.-%iger wäßriger Tetrafluoroborsäure versetzt. Man tropft 34,7 GT Natriumnitrit in 80 VT Wasser bei -2 bis 3°C zu. Nach zehnminütigem Rühren wird das ausgefallene Diazoniumsalz kalt abfiltriert und mit 300 VT Diethylether gewaschen. Man erhält nach Trocknung am Hochvakuum 111,0 GT eines gelben Diazoniumsalzes.
Ausbeute: 83 % 2-Chlor-4-N,N-dimethylaminophenyldiazoniumtetrafluoroborat
¹H-NMR: 3,32 (s, 6H, N(CH₃)₂, 7,25 (d, J = 9,0 Hz, 1H, CH), 8,30 (dd, J = 9,0, 2,5 Hz, 1H, CH), 8,51 (d, J = 2,5 Hz, 1H, CH).

### Beispiel 3 Reihenversuch zur Wiederverwendung des Katalysators

2,00 GT 2-Chlor-N,N-dimethyl-4-nitroanilin und 0,20 GT Palladium auf Aktivkohle (5 %ig, Fluka) werden in 6,0 VT Tetrahydrofuran suspendiert. Zu dem Reaktionsgemisch tropft man 0,94 GT einer 80 gew.- %igen wäßrigen Hydrazinlösung unter guter Kühlung so zu, daß die Innentemperatur zwischen 15 und 21°C bleibt. Anschließend wird zwei Stunden bei Raumtemperatur gerührt, der Katalysator abfiltriert und mit Tetrahydrofuran gewaschen. Das Filtrat wird nach Zusatz von 0,500 VT Tetradecan (Standard) gaschromatographisch auf Umsatz und Selektivität untersucht.
Der Katalysator wird nach dem Waschen mit Tetrahydrofuran luftgetrocknet und dann direkt zur nächsten Reduktion eingesetzt.
Ausbeute 94 % 3-Chlor-4-N,N-dimethylaminoanilin

### Versuch 2:

Ausbeute: 94 % 3-Chlor-4-N,N-dimethylaminoanilin

### Versuch 3:

Ausbeute: 90 % 3-Chlor-4-N,N-dimethylaminoanilin

### Versuch 4:

Ausbeute: 95 % 3-Chlor-4-N,N-dimethylaminoanilin

### Versuch 5:

Ausbeute: 92 % 3-Chlor-4-N,N-dimethylaminoanilin

### Versuch 6:

Ausbeute: 90 % 3-Chlor-4-N,N-dimethylaminoanilin

### Versuch 7:

Ausbeute: 99 % 3-Chlor-4-N,N-dimethylaminoanilin

### Versuch 8:

Ausbeute: 90 % 3-Chlor-4-N,N-dimethylaminoanilin

### Versuch 9:

Ausbeute: 92 % 3-Chlor-4-N,N-dimethylaminoanilin

### Versuch 10:

Ausbeute: 90 % 3-Chlor-4-N,N-dimethylaminoanilin

### Versuch 11:

Ausbeute: 91 % 3-Chlor-4-N,N-dimethylaminoanilin

### Beispiel 4 3-Chloranilin

3,15 GT 3-Chlornitrobenzol werden in 10 VT Tetrahydrofuran gelöst. Dazu gibt man 1 mol-% Palladium auf Aktivkohle (5 %ig) und 1,16 GT einer 80 gew.-%igen wäßrigen Hydrazinlösung so zu, daß die Temperatur 30°C nicht übersteigt. Anschließend wird eine Stunde bei Raumtemperatur gerührt. Die gaschromatographische Analyse des Reaktionsgemisches zeigt bei 99 % Umsatz 96 % Produkt und 3 % dechloriertes Anilin. Ausbeute: 96 % 3-Chloranilin

### Beispiel 5 4-Chlor-2-methyl-anilin

3,43 GT 4-Chlor-2-methyl-nitrobenzol werden in 10 VT Tetrahydrofuran gelöst. Dazu gibt man 1 mol-% Palladium auf Aktivkohle (5 %ig) und 1,16 GT einer 80 gew.-%igen wäßrigen Hydrazinlösung so zu, daß die Temperatur 20°C nicht übersteigt. Anschließend wird eine Stunde bei Raumtemperatur gerührt. Die gaschromatographische Analyse des Reaktionsgemisches zeigt bei 99 % Umsatz 94 % Produkt und 4 % dechloriertes Anilin. Ausbeute: 96 % 4-Chlor-2-methyl-anilin.

### Beispiel 6 2-Chlor-4-N,N, dimethylaminoanilin

Es wird wie in Beispiel 1 verfahren, jedoch verwendet man folgende Mengen und Bedingungen:
2,0 GT 2-Chlor-4-N,N-dimethylnitroanilin
6,0 VT Tetrahydrofuran
0,2 GT Palladium auf Aktivkohle (5 %ig)
0,92 VT 80 gew.-%ige wäßrige Hydrazinlösung bei 40°C Reaktionstemperatur.

Die gaschromatographische Analyse zeigt bei 99 % Umsatz 98 % Produkt und 1 % dechloriertes Anilin.

## Patentansprüche

1. Verfahren zur Herstellung halogenierter aromatischer Amine durch Reduktion der entsprechenden Nitroverbindungen mit Hydrazin in Gegenwart eines heterogenen Katalysators, der ein Metall aus der Gruppe VIIIa enthält, dadurch gekennzeichnet, daß die Umsetzung
a) in einem Temperaturbereich von etwa -20°C bis etwa 70°C mit
b) etwa 1,0 bis etwa 1,7 Äquivalenten Hydrazin, bezogen auf die zu reduzierende(n) Nitrogruppe(n),
c) in Gegenwart eines heterogenen Palladiumkatalysators,
durchgeführt wird.

2. Verfahren nach Anspruch 1, dadurch gekennzeichnet, daß die Umsetzung in einem Temperaturbereich von etwa 0°C bis etwa 50°C durchgeführt wird.

3. Verfahren nach einem Anspruch 1 oder 2, dadurch gekennzeichnet, daß der heterogene Palladiumkatalysator metallisches Palladium auf einem Trägermaterial ist.

4. Verfahren nach Anspruch 3, dadurch gekennzeichnet, daß das Trägermaterial Aktivkohle ist.

5. Verfahren nach einem oder mehreren der vorhergehenden Ansprüche, dadurch gekennzeichnet, daß als Ausgangsmaterial eine Verbindung der Formel I eingesetzt wird, 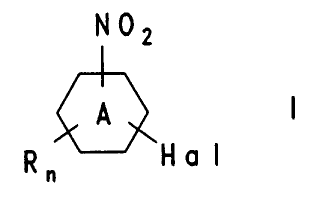 worin die Symbole und Indizes folgende Bedeutung haben: 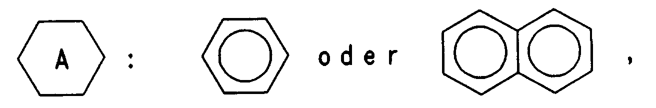
Hal: -F, -Cl, -Br, -I, vorzugsweise -Cl, -Br, -I, besonders bevorzugt -Cl, -Br,
n: je nach aromatischem Grundkörper 0, 1, 2, 3, 4 oder 0, 1, 2, 3, 4, 5, 6,
R: gleich oder verschieden -H, -F, -Cl, -Br, -I, CN, -SCN, -OCN, -NCO, -OH, -NH₂, -CHO, -NO₂, -SO₃H, R', OR', -COOR', -OCOR', -NHR', -NR'₂, -NA'H, -NA'R', -NA'₂, NHCOR', NHCOA',
R': gleich oder verschieden eine verzweigte oder unverzweigte Alkyl-, Alkenyl- oder Alkinylgruppe mit 1 bis 12, vorzugsweise 1 bis 8, Kohlenstoffatomen, wobei auch ein oder mehrere CH₂-Gruppen durch -O- und ein oder mehrere H-Atome durch -F, -Cl, -Br, -I, -OH und/oder -NH₂ ersetzt sein können,
A': Phenyl und/oder Naphthyl, die beide gegebenenfalls substituiert sein können.

6. Halogenierte aromatische Amine der Formel II 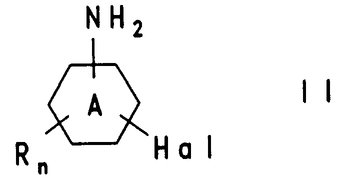 in der die Symbole und Indizes folgende Bedeutung haben: 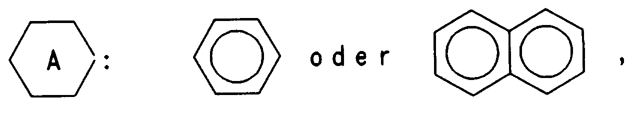
Hal: -F, -Cl, -Br, -I, vorzugsweise -Cl, -Br, -I, besonders bevorzugt -Cl, -Br,
n: je nach aromatischem Grundkörper 0, 1, 2, 3, 4 oder 0, 1, 2, 3, 4, 5, 6,
R: gleich oder verschieden -H, -F, -Cl, -Br, -I, CN, -SCN, -OCN, -NCO, -OH, -NH₂, -CHO, -SO₃H, R', OR', -COOR', -OCOR', -NHR', -NR'₂, -NA'H, -NA'R', -NA'₂, NHCOR', NHCOA',
R': gleich oder verschieden eine verzweigte oder unverzweigte Alkyl-, Alkenyl- oder Alkinylgruppe mit 1 bis 12, vorzugsweise 1 bis 8, Kohlenstoffatomen, wobei auch ein oder mehrere CH₂-Gruppen durch -O- und ein oder mehrere H-Atome durch -F, -Cl, -Br, -I, -OH und/oder -NH₂ ersetzt sein können,
A': Phenyl und/oder Naphthyl, die beide gegebenenfalls substituiert sein können,
hergestellt nach einem Verfahren nach einem oder mehreren der Ansprüche 1 bis 5.

7. Verwendung von Verbindungen, hergestellt mit einem Verfahren nach einem oder mehreren der Ansprüche 1 bis 5, als Zwischenprodukte zur Herstellung von Wirkstoffen, Polymeren und/oder Farbstoffen.

8. Verfahren zur Herstellung halogenierter aromatischer Diazoniumsalze, dadurch gekennzeichnet, daß
a) eine halogenierte aromatische Nitroverbindung, gegebenenfalls in einem Lösungsmittel, bei einer Temperatur zwischen etwa -20°C und etwa 70°C mit 1,0 bis 1,7 Äquivalenten Hydrazin, bezogen auf die zu reduzierende(n) Nitrogruppe(n), in Gegenwart eines heterogenen Palladiumkatalysators umgesetzt wird,
b) der Katalysator abfiltriert wird,
c) die Reaktionslösung bei -10°C bis 15°C mit einem Überschuß einer Säure und anschließend mit einer wäßrigen Lösung von Natriumnitrit versetzt wird,
d) gegebenenfalls nachgerührt wird und
e) das entstandene Diazoniumsalz abfiltriert wird.

9. Halogeniertes aromatisches Diazoniumsalz der Formel III 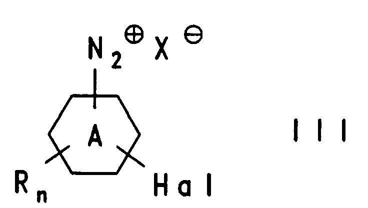 worin die Symbole und Indizes folgende Bedeutung haben: 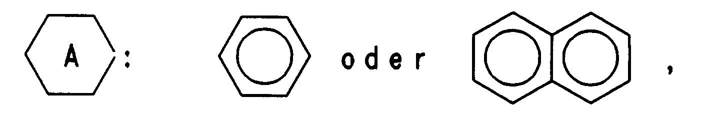
Hal: -F, -Cl, -Br, -I, vorzugsweise -Cl, -Br, -I, besonders bevorzugt -Cl, -Br,
n: je nach aromatischem Grundkörper 0, 1, 2, 3, 4 oder 0, 1, 2, 3, 4, 5, 6,
R: gleich oder verschieden -H, -F, -Cl, -Br, -I, CN, -SCN, -OCN, -NCO, -OH, -CHO, NO₂, -SO₃H, R', OR', -COOR', -OCOR', -NHR', -NR'₂, -NA'H, -NA'R', -NA'₂, NHCOR', NHCOA',
R': gleich oder verschieden eine verzweigte oder unverzweigte Alkyl-, Alkenyl- oder Alkinylgruppe mit 1 bis 12, vorzugsweise 1 bis 8, Kohlenstoffatomen, wobei auch ein oder mehrere CH₂-Gruppen durch -O- und ein oder mehrere H-Atome durch -F, -Cl, -Br, -I, -OH und/oder -NH₂ ersetzt sein können,
A': Phenyl und/oder Naphthyl, die beide gegebenenfalls substituiert sein können,
X: Cl, BF₄, PF₆, HSO₄, SO₄⁻, Tosylat, 5-Sulfosalicylat,
hergestellt mit einem Verfahren nach Anspruch 8.

10. Verwendung von halogenierten aromatischen Diazoniumsalzen, hergestellt mit einem Verfahren nach Anspruch 8, als Diazotypievorprodukte.
